(19) Europäisches Patentamt
European Patent Office
Office européen des brevets

(11) **EP 3 915 528 A1**

(12) **EUROPEAN PATENT APPLICATION**
published in accordance with Art. 153(4) EPC

(43) Date of publication:
**01.12.2021 Bulletin 2021/48**

(21) Application number: **20745911.6**

(22) Date of filing: **23.01.2020**

(51) Int Cl.:
*A61F 9/007* (2006.01)        *G02B 6/00* (2006.01)
*F21V 8/00* (2006.01)         *A61N 5/06* (2006.01)

(86) International application number:
**PCT/JP2020/002348**

(87) International publication number:
**WO 2020/153430 (30.07.2020 Gazette 2020/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.01.2019 JP 2019010835**

(71) Applicant: Santen Pharmaceutical Co., Ltd.
**Kita-ku
Osaka-shi
Osaka 530-8552 (JP)**

(72) Inventors:
• **KAMIKATANO, Mitsuru**
  **Sakura-shi, Chiba 285-8550 (JP)**
• **OKABE, Komei**
  **Ikoma-shi, Nara 630-0101 (JP)**
• **KOYAMA, Yukie**
  **Ikoma-shi, Nara 630-0101 (JP)**
• **OHASHI, Koji**
  **Ikoma-shi, Nara 630-0101 (JP)**

(74) Representative: **Vos, Derk**
**Maiwald Patentanwalts- und
Rechtsanwaltsgesellschaft mbH
Elisenhof
Elisenstraße 3
80335 München (DE)**

(54) **OPTICAL PROBE**

(57)  To prove an optical probe that can illuminate an entire desired region at one time, even in a narrow space. An optical probe including: a linear light guide to guide light from a light source device; a planar emitter to be connected to a tip portion of the linear light guide, to emit the light; a reflective surface provided in a part of the planar emitter, to reflect the light having entered the planar emitter; and an emitting surface provided so as to face the reflective surface, to allow the light reflected by the reflective surface to be emitted from a region of the reflective surface.

FIG. 3A

**Description**

[Technical Field]

The present invention relates to an optical probe.

[Background Art]

Typical optical probes each guide light from a light source device through a linear light guide such as an optical fiber or the like and emit the light from an end thereof. The light emitted from such an optical probe, however, is applied to a point, which prevents the optical probe from illuminating an entire desired region at one time. Accordingly, some optical probes are known to have an optical system, such as a lens and/or a mirror, on the distal end side thereof to control a region to be illuminated. PTL 1 discloses the provision of an endoscope and illumination device for endoscope capable of illuminating a subject with uniform illumination light without unevenness in brightness and color. PTL 2 discloses a light conductor integrated with an optical element made of a highly transmissive synthetic resin.

[Citation List]

[Patent Literature]

[PTL 1] Japanese Patent No.6309654
[PTL 2] Japanese Patent No.6336130

[Summary of Invention]

[Technical Problem]

Incidentally, there may be cases where an optical probe is inserted into a certain place to illuminate a desired region. Conventional optical probes each having an optical system such as a lens and/or a mirror, however, are limited with respect to where they can be inserted due to the size of the optical system. Accordingly, conventional optical probes cannot illuminate a desired region in a narrow space at one time.
The present invention is directed to provision of an optical probe that can illuminate an entire desired region at one time, even in a narrow space.

[Solution to Problem]

[Advantageous Effects of Invention]

A primary aspect of the present invention is an optical probe comprising: a linear light guide to guide light from a light source device; a planar emitter to be connected to a tip portion of the linear light guide, to emit the light; a reflective surface provided in a part of the planar emitter, to reflect the light having entered the planar emitter; and an emitting surface provided so as to face the reflective surface, to allow the light reflected by the reflective surface to be emitted from a region of the reflective surface. Other features of the present invention will become apparent from the description in the present specification and the accompanying drawings.
According to the present invention, a desired region can be illuminated at one time even in a narrow space.

[Brief Description of Drawings]

Fig. 1 is a schematic diagram illustrating an overall structure of a lighting system according to an embodiment of the present invention.
Fig. 2 is a schematic diagram explaining an optical probe according to an embodiment of the present invention.
Fig. 3A is a diagram explaining an optical probe according to an embodiment of the present invention.
Fig. 3B is a diagram explaining an optical probe according to an embodiment of the present invention.
Fig. 4 is a diagram explaining an optical probe according to a modified example 1.

Fig. 5A is a diagram explaining an optical probe according to a modified example 2.
Fig. 5B is a diagram explaining an optical probe according to a modified example 2.
Fig. 6A is a diagram explaining an optical probe according to a modified example 3.
Fig. 6B is a diagram explaining an optical probe according to a modified example 3.
Fig. 7A is a diagram explaining a planar emitter used for optical simulations.
Fig. 7B is a diagram explaining a planar emitter used for optical simulations.
Fig. 8A is a diagram explaining a planar emitter used for optical simulations.
Fig. 8B is a diagram explaining a planar emitter used for optical simulations.
Fig. 8C is a diagram explaining a planar emitter used for optical simulations.
Fig. 9 gives results of optical simulations.
Fig. 10 is a diagram explaining test results with respect to scleral reinforcement effects.
Fig. 11 is a diagram explaining test results with respect to a cross-linking rate of collagen.

[Description of Embodiments]

<Embodiments>

== Overview of lighting system ==

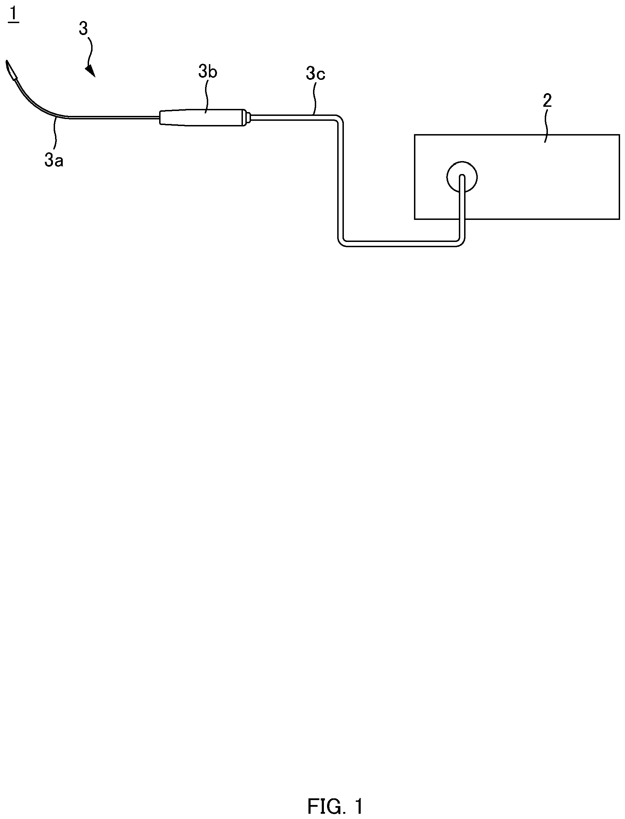

Fig. 1 is a schematic diagram illustrating an overall structure of a lighting system 1 according to an embodiment of the present invention. The lighting system 1 includes a light source device 2 and an optical probe 3. The optical probe 3 is a probe for illumination with light. The optical probe 3 includes an insertion portion 3a, a handle portion 3b, a cable portion 3c, and the like (details will be described later). The light source device 2 has a light source such as a halogen lamp that generates light. The light source device 2 supplies light to the optical probe 3.

== Optical probe ==

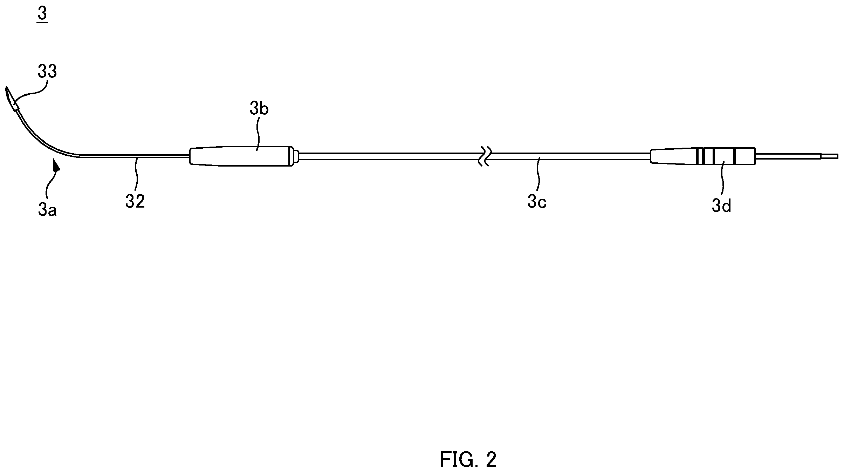

Fig. 2 is a schematic diagram explaining a structure of the optical probe 3 according to an embodiment of the present invention.

The insertion portion 3a is a portion to be inserted into a site to be illuminated with light. The insertion portion 3a comprises a linear light guide 32 and a planar emitter 33.

The linear light guide 32 is a member to guide light from the light source device 2 to the planar emitter 33. A tip portion of the linear light guide 32 is coupled to the planar emitter 33. A base portion of the linear light guide 32 extends through the handle portion 3b and the cable portion 3c to a light guide plug 3d.

The linear light guide 32 is formed of, for example, an optical fiber or an optical fiber bundle. The linear light guide 32 is encased in an outer conduit (not shown). The outer conduit is made of, for example, a metal such as stainless steel.

The planar emitter 33 is a member that emits the light having entered from the linear light guide 32. The planar emitter 33 may be made of, for example, a transparent material such as acrylic resin and/or the like. The planar emitter 33 is provided at a tip portion of the insertion portion 3a. A structure of the planar emitter 33 will be described later.

The handle portion 3b is a portion to be held by a user when using the optical probe 3. The handle portion 3b is coupled, at the tip portion side thereof, to a base portion of the insertion portion 3a. The handle portion 3b is coupled, at the base portion side thereof, to a tip portion of the cable portion 3c.

The cable portion 3c is a member to connect the optical probe 3 to the light source device 2. The cable portion 3c is a hollow member made of, for example, resin and/or the like, and allows the linear light guide 32 extending from the insertion portion 3a to be inserted thereinto. The light guide plug 3d is provided at a base portion of the cable portion 3c. The base portion of the linear light guide 32 is disposed at the light guide plug 3d. By connecting the light guide plug 3d to the light source device 2, the light from the light source device 2 can be supplied to the linear light guide 32 and emitted from the tip side of the insertion portion 3a (planar emitter 33).

== Details of planar emitter ==

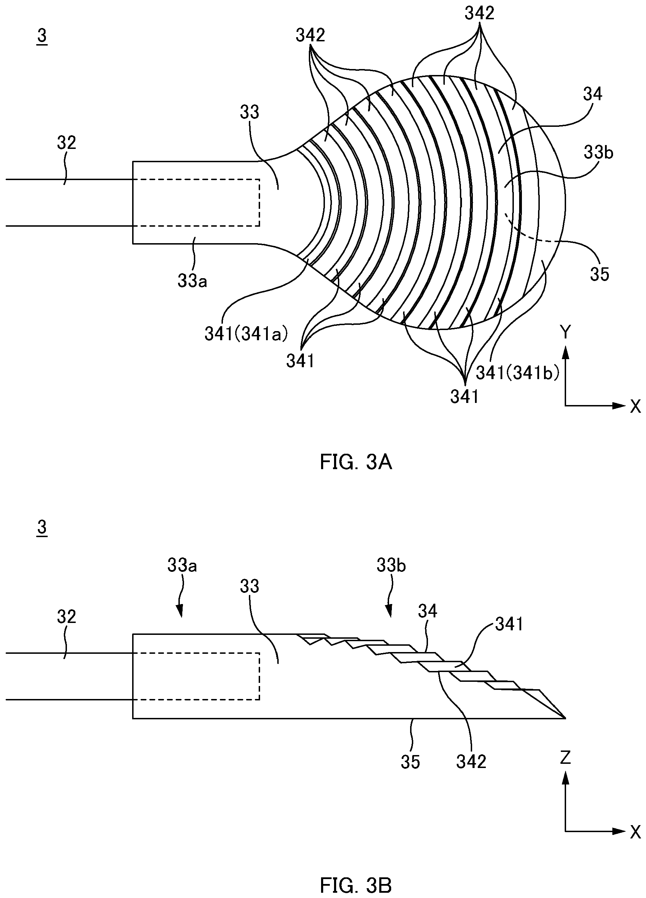

Next, a structure of the planar emitter 33 will be described in detail with reference to Figs. 3A and 3B. In the following, a Cartesian coordinate system consisting of the following X, Y, and Z-directions is used herein. It is assumed that a plane parallel to an emitting surface 35 corresponds to an XY plane. In the XY plane, the longitudinal direction of the planar emitter 33 corresponds to the X-direction, and the direction perpendicular to the X-direction corresponds to the Y-direction. The direction perpendicular to the emitting surface 35 corresponds to the Z-direction. Fig. 3A is a plan view of the planar emitter 33 according to an embodiment of the present invention viewed from above (from the reflective surface 34 side) in the Z-direction. Fig. 3B is a side view of the planar emitter 33 viewed from the Y-direction.

The planar emitter 33 has a connecter portion 33a and a disk portion 33b. The connecter portion 33a is a portion to be connected to the linear light guide 32. As illustrated in Fig. 3A, the connecter portion 33a extends from the disk portion 33b. The tip portion of the linear light guide 32 is fitted into the connecter portion 33a. The light emitted from the tip portion of the linear light guide 32 enters the disk portion 33b through the connecter portion 33a. The disk portion 33b is a disk-shaped portion. The disk portion 33b has a predetermined thickness in the Z-direction. When viewed from the Z-direction, the disk portion 33b is substantially circular. The disk portion 33b has the reflective surface 34 and the emitting surface 35.

The reflective surface 34 reflects the light having entered the planar emitter 33. The light having entered the planar emitter 33 (disk portion 33b) is dispersed in multiple directions in the disk portion 33b, and each dispersed component of the light reaches the reflective surface 34. The reflective surface 34 is configured to reflect these components of the light toward the emitting surface 35.

Specifically, in an embodiment of the present invention, the reflective surface 34 is in a form of a staircase including a plurality of step portions 341 and a plurality of flat portions 342 each provided between the step portions 341 adjacent thereto (see Fig. 3B). The step portions 341 and the flat portions 342 are alternately continuous. Each of the step portions 341 has an angle with respect to the emitting surface 35. This angle corresponds to an angle between a tangent plane at a given point on a step portion 341 of the reflective surface 34 and the emitting surface 35. In other words, the tangent plane is not parallel to the emitting surface 35, and the angle formed between the tangent plane and the emitting surface 35 is greater than 0 degrees. The angle of each of the step portions 341 with respect to the emitting surface 35 is preferably 25 degrees or more and 65 degrees or less. The flat portions 342 are substantially parallel to the emitting surface 35. In other words, the tangent plane at a given point on a flat portion 342 of the reflective surface 34 and the emitting surface 35 are substantially parallel to each other.

As illustrated in Fig. 3A, in an embodiment of the present invention, the step portions 341 and the flat portions 342 are provided concentrically. In an embodiment of the present invention, the step portions 341 and the flat portions 342 are provided concentrically around a point near the tip portion of the linear light guide 32. In an embodiment of the present invention, nine step portions 341 are provided (see Fig. 3A). The distances each between the step portions 341 adjacent to each other (i.e., the widths of flat portions 342) are almost equal. In the disk portion 33b, the innermost step portion 341 (341a) is closer to the tip portion of the linear light guide 32, and the outermost step 341 (341b) is closer to the tip portion of the disk portion 33b (on the side opposite to the side connected to the connecter portion 33a). Note that the arrangement of the step portions 341 according to an embodiment of the present invention is an example and is not limited thereto.

The emitting surface 35 is provided at a position opposite to the reflective surface 34 in the disk portion 33b. The emitting surface 35 allows the light reflected by the reflective surface 34 to be emitted from a region of the emitting surface 35 so as to illuminate a plane. When the light reflected from the reflective surface 34 arrives at the emitting surface 35, the light passes through the emitting surface 35 and is emitted to the outside.

As described above, the reflective surface 34 is configured such that the reflected light is directed toward the emitting surface 35, in the entire region of the reflective surface 34. Thus, the light reflected from the reflective surface 34 reaches multiple positions on the emitting surface 35. Accordingly, the emitting surface 35 is able to allow the light to be emitted from a region thereof.

In the disk portion 33b, the width between the reflective surface 34 and the emitting surface 35 (i.e., the distance between the reflective surface 34 and the emitting surface 35 in the Z-direction) decreases with distance from the connecter portion 33a (i.e., with distance from the tip portion of the linear light guide 32) (see Fig. 3B). In other words, the thickness of the disk portion 33b decreases with distance from the connecter portion 33a.

Note that when the planar emitter 33 is made of a transparent material, a portion of the light arriving at the reflective surface 34 passes through the reflective surface 34. To prevent such transmission of the light, a reflective film may be formed on the reflective surface 34. The reflective film is made of, for example, a metal such as aluminum, silver, or the like. The reflective film is formed by vapor deposition of metal. The light arriving at the reflective surface 34 having the reflective film formed thereon is reflected toward the emitting surface 35 without passing through the reflective surface 34.

== Examples of use of optical probe ==

The optical probe 3 according to an embodiment of the present invention can be used, for example, for a site in an organism. The site in an organism is, for example, a narrow site such as between the eyeball and the inner surface of the eyelid, or a stenosis due to a lesion or other abnormality. The planar emitter 33 of the optical probe 3 according to an embodiment of the present invention is disk-shaped and thus can be inserted into such a place. In addition, since the planar emitter 33 has the emitting surface 35 in a substantially circular shape, the light emitted from the emitting surface 35 is applied while spreading over a certain region. This means that the optical probe 3 can illuminate a desired region with the light at one time.

For affected parts in a living body, some approaches include administering, to a body, an agent that selectively reacts to light of specific wavelengths and directly illuminating the affected part in order to induce a chemical reaction of the agent in the body. In such cases, even when the affected part is in a narrow space or the like, the use of the optical probe 3 according to an embodiment of the present invention makes it possible to illuminate a desired region. Furthermore, with the optical probe 3 according to an embodiment of the present invention, it is possible to illuminate a predetermined region at one time, thereby being able to reduce the number of irradiation as compared with that when using a conventional optical probe, even in the case where the affected part is large.

Note that, in such cases, it is preferable that the light emitted from the planar emitter 33 is light to induce a chemical reaction of the agent. The light emitted from the planar emitter 33 may have any wavelength as long as it does not significantly affect the safety of organisms. For example, when the agent to be administered to a living body contains a photosensitive substance (photosensitizer) that is to be activated with ultraviolet light, the light emitted from the planar emitter 33 is preferably ultraviolet light. Likewise, when the agent to be administered to a living body contains a photo-sensitive substance (photosensitizer) that is to be activated with blue light, the light emitted from the planar emitter 33 is preferably blue light. Illumination of a site in an organism in which the agent containing the photosensitive substance is to be administered enables a selective treatment of the site. The photosensitive substance can be any compound as long as it is excited by light, and examples thereof include, for example, riboflavin, flavin mononucleotide (FMN), flavin adenine dinucleotide (FAD), and salts or hydrates thereof, and the like.

The site in an organism may be, for example, the sclera of the eye, preferably the posterior sclera. In the field of ophthalmology, if the rigidity of the sclera can be increased, the axial elongation of the eye can be reduced, and medical conditions such as intense myopia and staphyloma may be treated, alleviated or prevented (Japanese Patent No. 5487470). In this case, the planar emitter 33 of the optical probe 3 is inserted between the inner surface of the eyelid and the sclera. At this time, while maintaining the contact between the emitting surface 35 and the sclera, the planar emitter 33 is inserted thereinto along the periphery (surface) of the eyeball to a desired position (e.g., equator, posterior pole (or "back"), etc.).

After the planar emitter 33 is placed at a desired position on the sclera in this manner, illumination is performed. In the optical probe 3 according to an embodiment of the present invention, light is emitted from a region of the emitting surface 35. Accordingly, with the optical probe 3 according to an embodiment of the present invention, a predetermined region on the sclera (i.e., the region corresponding to the emitting surface 35) can be illuminated at one time.

For example, when treating the sclera of the eye, the optical probe 3 is inserted along the periphery of the eyeball up to the equator, and ultraviolet light is emitted from the planar emitter 33 while administering an agent containing riboflavin, which is one of the photosensitive substances. A chemical reaction is induced by the riboflavin and the ultraviolet light, thereby being able to improve the rigidity of the sclera in the illuminated region. The expression "chemical reaction is induced" in this case means, for example, cross-linking of collagen fibrils of the sclera by reactive oxygen species (e.g., singlet oxygen, superoxide, hydroxyl radical, etc.) generated by riboflavin and ultraviolet light. The cross-linking of collagen fibrils can increases the rigidity of the sclera.

The optical probe 3, when used for a site in an organism, may be reused by being cleaned and sterilized after use. However, it is preferable that the optical probe 3 is disposable from the viewpoint of preventing infection.

Fig. 3B illustrates an aspect in which the emitting surface 35 is a flat surface, however, it is not limited thereto. The emitting surface 35 may be designed as appropriate depending on the application. In the case of this example, the emitting surface 35 may be a curved surface that follows the surface of the sclera.

The lighting system 1, the optical probe 3, and examples of their use according to an embodiment of the present invention have been described, but the use of the optical probe 3 according to an embodiment of the present invention is not limited to the illumination of the sclera. The optical probe 3 can also be used, for example, for other organs in the living body. Moreover, it is not limited to the illumination of sites in an organism. The optical probe 3 can also be used, for example, for cracks in buildings, the interior of electronic devices, and the like. The size of the insertion portion 3a is not particularly limited, however, it is preferable that the size thereof is smaller when it is used by being inserted into a site in an organism. For example, the diameter of the disk portion of the planar emitter is preferably 20 mm or less, and more preferably, 10 mm or less. The longitudinal length of the planar emitter is preferably 30 mm or less, and more preferably, 20 mm or less. The maximum thickness of the planar emitter is preferably 10 mm or less, and more preferably, 5 mm or less.

As apparent from hereinabove, the optical probe 3 according to an embodiment of the present invention comprises: a linear light guide 32 to guide light from the light source device 2; the planar emitter 33 to be connected to the tip portion of the linear light guide 32, to emit the light; the reflective surface 34 provided in a part of the planar emitter 33, to reflect the light having entered the planar emitter 33; and the emitting surface 35 provided so as to face the reflective surface 34, to allow the light reflected by the reflective surface 34 to be emitted from a region of the emitting surface 35.

With such a structure, the optical probe 3 according to an embodiment of the present invention can cause the light guided through the linear light guide 32 to be emitted from a region of the emitting surface 35 to illuminate a plane. Accordingly, the optical probe 3 does not need, at its distal end, such an optical system as a lens, a mirror, and/or the like to control a range of illumination. This enables users to use the optical probe 3 in a narrow space such as between the inner surface of the eyelid and the eyeball. In addition, since the light is emitted in a plane from a region of the emitting surface 35, a single irradiation is enough to illuminate a desired region. Thus, as compared to conventional optical probes, it is possible to illuminate a wide region with a single irradiation. That is, the optical probe 3 according to an embodiment of the present invention can illuminate the entire desired region at one time, even in a narrow space.

Furthermore, in the planar emitter 33 of the optical probe 3 according to an embodiment of the present invention, a distance between the emitting surface 35 and the reflective surface 34 in the planar emitter 33 decreases with distance from the tip portion of the linear light guide 32. In a configuration in which the distance increases with distance therefrom or is approximately constant, it is difficult to make the intensity of the emitted light uniform because the light become weaker as it travels from the tip portion of the linear light guide 32. This makes it possible to efficiently emit light from the emitting surface 35 and improve the uniformity of the intensity of the emitted light.

Moreover, the reflective surface 34 of the optical probe 3 according to an embodiment of the present invention includes: a plurality of step portions 341 each having an angle with respect to the emitting surface 35; and a plurality of flat portions 342 each provided between the step portions 341 adjacent to each other of the plurality of step portions, and is in a form of a staircase in which the plurality of step portions 341 and the plurality of flat portions 342 are alternately continuous. By designing the angle and width of the step portions 341 and the width of the flat portions 342 at each position, it becomes possible to adjust the direction and intensity distribution of the light emitted from the emitting surface 35. This enables the light to be emitted from the emitting surface 35 more efficiently, and the uniformity of the intensity of the emitted light is further improved.

Furthermore, in the optical probe 3 according to an embodiment of the present invention, the plurality of step portions 341 each have an angle with respect to the emitting surface 35 of 25 degrees or more and 65 degrees or less. Accordingly, it becomes possible to reflect, at the step portions 341, the light having entered the planar emitter 33 from the linear light guide 32 and arrived at the step portions 341, to be efficiently emitted from the emitter surface 35. When the angle is too small, the light reflected by the step portions 341 is reflected again by the emitting surface and travels back inside the planar emitter 33. When the angle is too large, the light passes through the step portions 341 without being reflected thereby. This enables the light to be emitted from the emitting surface 35 more efficiently, and the uniformity of the intensity of the emitted light is further improved.

Moreover, in the optical probe 3 according to an embodiment of the present invention, the step portions 341 are provided concentrically. Accordingly, in a plan view, the step portions 341 face almost squarely the light having entered the planar emitter 33 from the linear light guide 32, thereby facilitating that the light reflected from the step portions 341 is incident onto the emitter surface 35 at an angle closer to the right angle with respect to the emitter surface 35. This enables the light to be emitted from the emitting surface 35 more efficiently, and the uniformity of the intensity of the emitted light is further improved.

Furthermore, in the optical probe 3 according to an embodiment of the present invention, the planar emitter 33 is substantially disk-shaped. Such a configuration facilitates the insertion of the optical probe 3 into a narrow space. In addition, when the optical probe 3 having such a configuration is used for a site in an organism, the site is less likely to be damaged.

In the optical probe 3 according to an embodiment of the present invention, the reflective surface 34 may have a reflective film formed thereon. This increases the reflectance of the reflective surface 34 with respect to the light having entered the planar emitter 33 from the linear light guide 32. Accordingly, the intensity of the light emitted from the emitting surface 35 is further increased. This makes it possible to emit the light more efficiently.

Moreover, in the optical probe 3 according to an embodiment of the present invention, the linear light guide 32 is an optical fiber or an optical fiber bundle. This can reduce the loss of light when the light from the light source device 2 is guided through the linear light guide 32.

Furthermore, in the optical probe 3 according to an embodiment of the present invention, an illuminated site is a site in an organism. In other words, the optical probe 3 can be inserted into a small part or a narrowed part in a living body, and a desired region can be illuminated.

Furthermore, in the optical probe 3 according to an embodiment of the present invention, the light is ultraviolet light to treat the site to which a photo-sensitive substance is administered, in an organism. This makes it possible to selectively treat only a region, in the site in an organism, to be irradiated with the ultraviolet light, in which a certain amount or more of the photo-sensitive substance is present.

Moreover, in the optical probe 3 according to an embodiment of the present invention, the site in an organism is an eyeball, and the emitting surface 35 has a predetermined surface area such that the emitting surface 35 can be placed along a sclera of the eyeball. Accordingly, with the emitting surface 35 fixedly placed along the sclera, a region facing the emitting surface 35 in the sclera can be illuminated at one time. This can reduce the time for inducing a chemical reaction over the desired region in the eyeball.

Some modified examples of an embodiment of the present invention will be described below.

<Modified example 1>

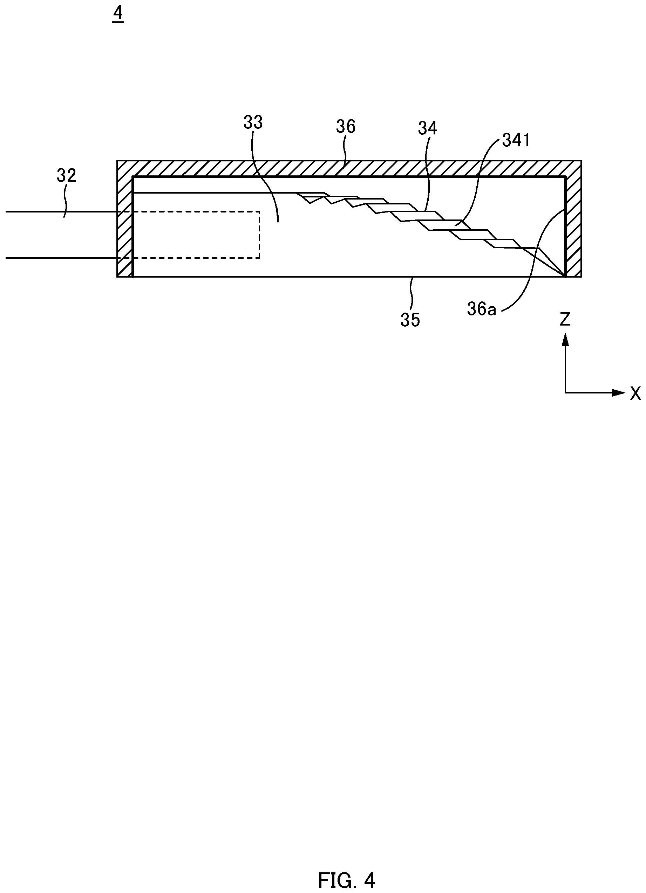

Fig. 4 is a diagram explaining a tip portion and its surroundings of an optical probe 4 in a modified example of an embodiment of the present invention. In a modified example, a reflective structure 36 is provided for the planar emitter 33. The reflective structure 36 reflects the light having passed through the reflective surface 34 to reenter the reflected light into the planar emitter 33. The reflective structure 36 is a container-shaped structure that covers the planar emitter 33, and a part thereof corresponding to the emitting surface 35 is open. The inner surface of the reflective structure 36 has been subjected to a reflective treatment at least on an inner surface 36a thereof facing the reflective surface 34. The reflective treatment increases the light reflectance. The reflective treatment may be performed by depositing a metal. Examples of the metal include materials similar to those of the reflective film described in an embodiment of the present invention. In the example of Fig. 4, the light having passed through the reflective surface 34 is reflected by the inner surface 36a. The light having been reflected from the inner surface 36a passes through the reflective surface 34 and reenters the planar emitter 33.

The reflective structure 36 may be made of any material, such as a metal, resin, and/or the like as long as the material is not restricted for use in a living body.

Instead of performing a reflective treatment as described above to the reflective structure 36, the reflective structure 36 itself may be made of a material having a desired reflectance. In this case, examples of the material of the reflective structure 36 include, for example, aluminum, stainless steel (SUS), and a similar metal.

<Modified example 2>

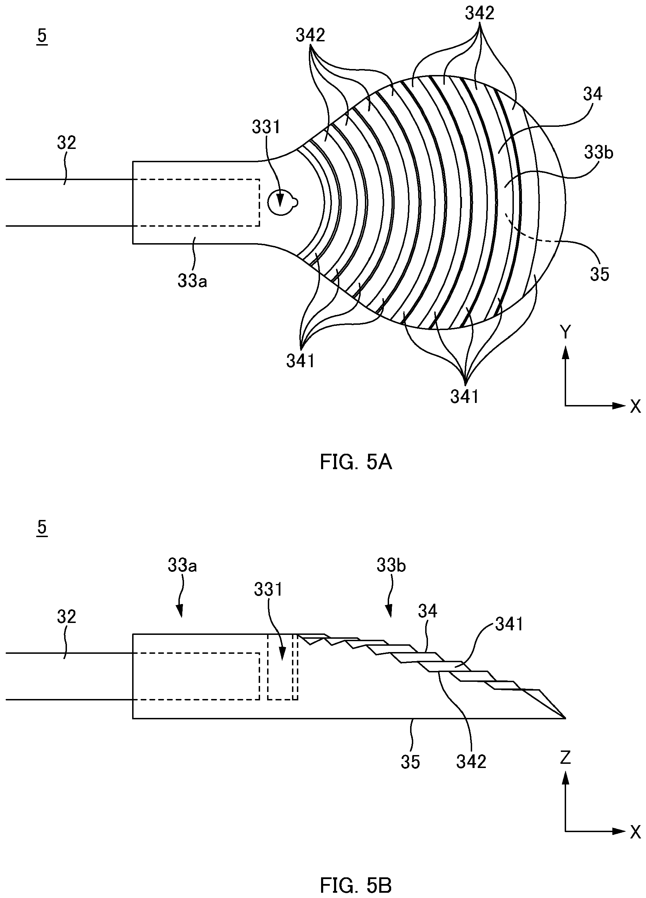

Figs. 5A and 5B are diagrams explaining a tip portion and its surroundings of an optical probe 5 in a modified example of an embodiment of the present invention. In a modified example, the planar emitter 33 includes a region 331 with a refractive index different from a refractive index or refractive indexes of the planar emitter 33 other than the region 331, the region 331 being provided on an optical path between a position at which the tip portion of the linear light guide 32 is to be connected to the planar emitter 33 and the reflective surface 34. The region 331 is provided to further disperse, in multiple directions, the light having entered the planar emitter 33 from the linear light guide 32.

In this modified example, the region 331 is provided near the position at which the tip portion of the linear light guide 32 is to be connected (see Fig. 5A). As illustrated in Fig. 5B, the region 331 is a cylindrical region (cavity) extending along the Z-direction. The end of the region 331 on the reflective surface 34 side is released. The region 331 has such a circular cross-sectional shape along the XY plane that a circle has a part protruding toward the disk portion 33b. Such a shape with a protrusion makes it possible to further disperse the incident light in multiple directions.

Note that the shape of the region 331 is not limited to the cylindrical shape as mentioned above. This modified example describes an aspect in which the region 331 is hollow, however, the region is not limited thereto. For example, as the region with a different refractive index, a material with a refractive index different from a refractive index of other region may be embedded.

<Modified example 3>

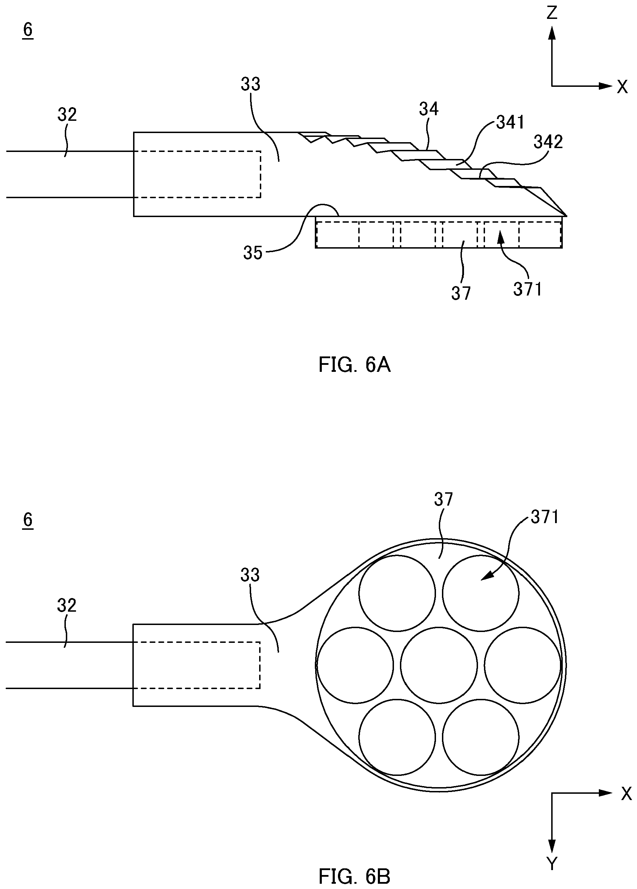

Figs. 6A and 6B are diagrams explaining a tip portion and its surroundings of an optical probe 6 in a modified example of an embodiment of the present invention. Fig. 6A is a side view of the optical probe 6 when viewed from the Y-direction. Fig. 6B is a plan view of the optical probe 6 when viewed from below (on the emitting surface 35 side) in the Z-direction. The planar emitter 33 of this modified example has a protrusion 37 protruding downward in the Z-direction from the emitting surface 35. The protrusion 37 has a plurality of holes 371. Each of the holes 371 is a recessed hole communicating with the outside. Each of the holes 371 may not be a recessed hole, but be a through-hole and open to the emitting surface 35.

In this modified example, the protrusion 37 has seven holes 371. Each of the seven holes 371 is a cylindrical hole 371 having a circular cross section. Note that the number and shape of the holes 371 are not limited thereto. The holes 371 can hold air thereinside. The amount of air allowed to be held therein can be increased or decreased by changing one or more of the diameter of the holes 371, the number of holes 371, and the depth (length along the Z-direction) of the holes 371.

The protrusion 37 is formed integrally with the planar emitter 33. Alternatively, the protrusion 37 may be formed as a separate component and then joined to the emitting surface 35 of the planar emitter 33. Each of the holes 371 may be subjected to a reflective treatment on their side surfaces. With such a reflective treatment, the light emitted from the emitting surface 35 can be efficiently applied for illumination.

When the administration of an agent and the illumination of light as described above are performed using the optical probe 6, it is preferable to supply oxygen to induce a chemical reaction between the agent and the affected part of the living body. For example, in the example of use for the eyeball described above, when the optical probe 6 according to this modified example is inserted between the eyeball and the inner surface of the eyelid, the protrusion 37 comes into contact with the sclera. As a result, the openings of the holes 371 are closed by the sclera, and thus a closed space is formed in each hole 371. Such a closed space has air held therein. In other words, with the provision of the protrusion 37, it is possible to insert the optical probe 6 into a site in an organism with the air held in the holes 371. When illumination is performed in this state, a chemical reaction is further induced by the oxygen by virtue of the air held in the holes 371.

As apparent from the above, in the optical probe 4 according to the modified example 1, the reflective structure 36 covers the reflective surface 34 and has a surface facing the reflective surface 34. The surface facing the reflective surface 34 has been subjected to a reflective treatment, to reflect the light having passed through the reflective surface 34, toward the inside of the planar emitter 33. The light that has entered the planar emitter 33 from the linear light guide 32 and then passed through the reflective surface 34 to be emitted outside the planer emitter 33, can be reentered into the planar emitter 33. Then, the planar emitter 33 can emit, from the emitting surface 35, the light having reentered into the planar emitter 33. Accordingly, the intensity of the light emitted from the emitting surface 35 further increases. This makes it possible to emit the light even more efficiently.

In the optical probe 5 according to the modified example 2, the planar emitter 33 includes the region 331 with a refractive index different from a refractive index or refractive indexes of the planar emitter 33 other than the region 331, the region 331 being provided on an optical path between a position at which the tip portion of the linear light guide 32 is to be connected to the planar emitter 33 and the reflective surface 34. The light having entered the planar emitter 33 from the linear light guide 32 is further dispersed in multiple directions by being guided through the region 331. By being further dispersed, the light is more likely to be reflected over the entire region of the reflective surface 34. This improves the uniformity of the intensity of the light emitted from the emitting surface 35.

Furthermore, in the optical probe according to a modified example, the light is light to induce a chemical reaction at the site in an organism. With the optical probe according to an embodiment of the present invention, it is possible to illuminate a desired region in a desired site of a living body at one time, thereby being able to induce the chemical reaction simultaneously over the desired region. This can reduce the time for the chemical reaction to be induced over the desired region. In addition, with the optical probe 4 according to the modified example 1, it is possible to emit the light more efficiently from the emitting surface 35, thereby being able to further reduce the time for the chemical reaction. Moreover, with the optical probe 5 according to the modified example 2, the uniformity of the intensity of the light emitted from the emitting surface 35 is further improved, thereby being able to induce the chemical reaction even more uniformly over the desired region.

The optical probe 6 according to the modified example 4 includes the protrusion 37 protruding from the emitting surface 35. The protrusion 37 has the plurality of holes 371 communicating with the outside. The holes 371 can be allowed to include the air therein. This causes the air in the holes 371 to further induce the chemical reaction.

[Examples]

<Optical Simulations>

Optical simulations were performed to examine effects on the uniformity of the intensity of the emitted light when a region with a different refractive index is provided on an optical path, as in the region 331 with a different refractive index described in the modified example 3. The simulation software used was Zemax OpticStudio from Zemax. The conditions for each example are as follows.

(Example 1)

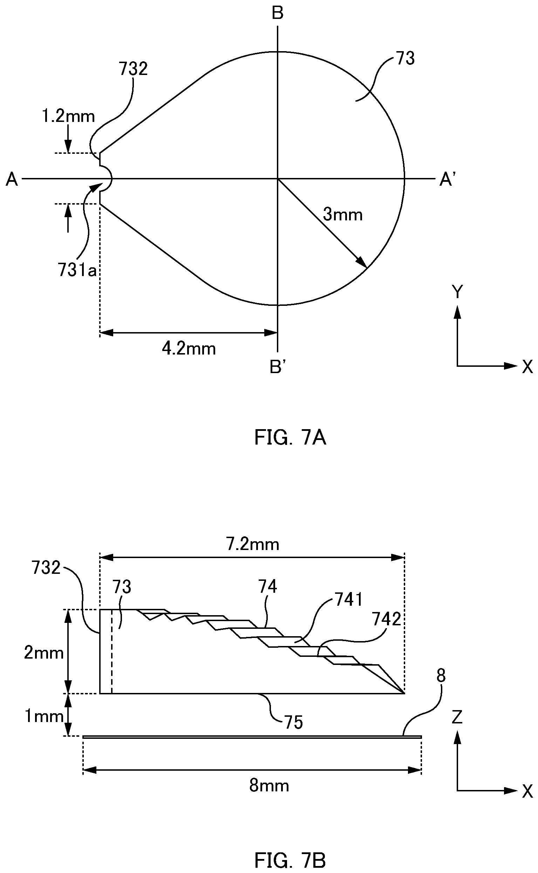

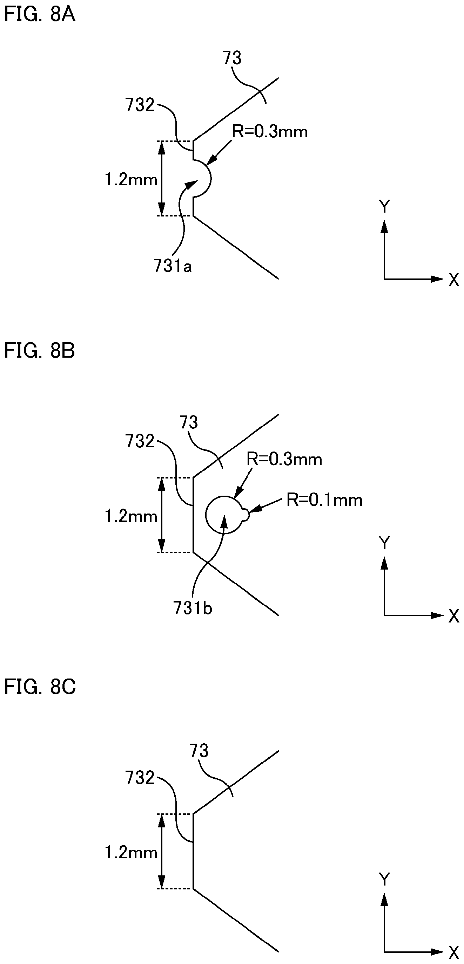

Figs. 7A, 7B and 8A are diagrams explaining a structure of a planar emitter 73 of Example 1. Fig. 8A is a diagram explaining a shape of a light incident surface 732 and its surroundings. In Example 1, a region 731a with a different refractive index is provided. The region 731a in Example 1 is a recess that is formed in the light incident surface 732 and extends along the Z-direction. The recess has a semicircular cross section with a radius of 0.3 mm (Fig. 8A). As illustrated in these figures, the flat light incident surface 732 parallel to the YZ plane was provided in the planar emitter 73. In this simulation, light is caused to enter the planar emitter 73 in the direction perpendicular to the light incident surface 732 (i.e., in the X direction).

(Example 2)

In Example 2, the shape of a region 731b with a different refractive index differs from that of Example 1. The region 731b in Example 2 is a cylindrical region that is provided near the light incident surface 732 and extends along the Z-direction. The cross-sectional shape of the cylindrical region corresponds to a contour obtained by combining two circles with radii of 0.3 mm and 0.1 mm such that a part of one circle is superimposed on a part of the other circle (Fig. 8B).

(Comparative Example 1)

Comparative Example 1 differs from Examples 1 and 2 in that the region 731a with a different refractive index is not provided (Fig. 8C).

(Common conditions)

The following describes the conditions that Examples 1 and 2, and Comparative Example 1 share.

[Light source]

An optical fiber with a radius of 1 mm was coupled to the light incident surface 732 of the planar emitter 73. The intensity of the light traveling through the optical fiber was set to 1 W, and the light was caused to enter the planar emitter 73.

[Planar emitter]

The refractive index of the planar emitter 73 was set to 1.49. The reflectance of the surfaces other than the light incident surface 732 and the emitting surface 75 was set to 95%. The refractive index was set to 1.35 in the region outside the planar emitter 73 and below the emitting surface 75.

[Photodetector]

A photodetector 8 was placed to measure the intensity distribution of the light emitted from the emitting surface 75. The photodetector 8 was 8 mm square and was placed at a position 1 mm below the emitting surface 75 (Fig. 7B).

(Simulation results)

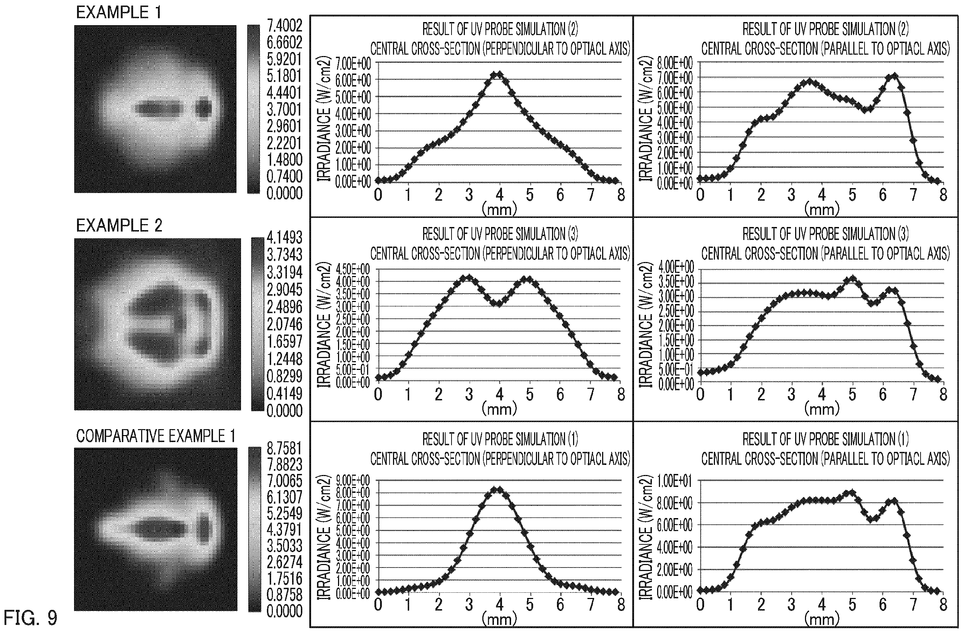

The results of the optical simulations are given in Fig. 9. The top, middle, and bottom rows of Fig. 9 give the results of Example 1, Example 2, and Comparative Example 1, respectively. In each result, the left figure gives the intensity distribution on the XY plane detected by the photodetector 8. The middle and right figures give the intensity distributions corresponding to lines B-B' and A-A' illustrated in Fig. 7A respectively.

The light-receiving efficiency detected by the photodetector 8 was 88%, 84%, and 89% in Example 1, Example 2, and Comparative Example 1, respectively.

When comparing the intensity distributions among the examples, it can be understood that Examples 1 and 2, which include the region with a different refractive index, have a higher uniformity of light intensity than Comparative Example 1, which includes no such region. These results indicated that the uniformity of the intensity of the light emitted from a region of the emitting surface 75 is improved by providing the region with a different refractive index.

<Increasing scleral rigidity>

In the field of ophthalmology, if the rigidity of the sclera can be increased by cross-linking of collagen fibrils, the axial elongation of the eye may be reduced, and medical conditions such as intense myopia and pathological myopia may be treated, alleviated or prevented. In fact, the following tests were conducted to demonstrate the effects of chemical reactions between photosensitive substances and ultraviolet light emitted from optical probes on tissues of an organism.

(Test method and measurement method)

The tests were conducted in accordance with a method described in the scientific literature "J Cataract Refract Surg., 2004, 30(3), 689-695 as follows.

Step 1) Rabbit scleral strips of 10 mm in length and 5 mm in width were immersed in 0.2% riboflavin-5'-phosphate solution for 15 minutes.

Step 2) The scleral strips were exposed to ultraviolet light (wavelength: 365 nm, intensity: 3 mW/cm$^2$) for 30 minutes using an optical probe and a light source device (Panasonic ANUJ3500). This test used an optical probe comprising a planar emitter of 10 mm in longitudinal length and 2 mm in maximum thickness, with a disk portion thereof of 6 mm in diameter. A hollow region was provided as the region with a different refractive index near the position where the tip portion of the linear light guide is connected to the planar emitter. The region was cylindrical in shape, extending along the thickness of the planar emitter and penetrating the planar emitter. The region has a circular cross-sectional shape along a plane parallel to the emitting surface, with a part of the circle protruding toward the disk portion.

Step 3) Each scleral strip was clamped to the EZ Test (small tabletop testing machine, Shimadzu Corporation, model: EZ-S) at a distance of 2 mm between jaws. After applying a preload of 0.1 N for 1 minute, the strip was pulled at a speed of 1 mm/min, and the tension (N) at a displacement of 0.8 mm was taken as a measure of the scleral rigidity.

The samples subjected to the above steps 1) to 3) were taken as Example 3, and those subjected to only the step 3) were taken as Comparative Example 2. Three trials were conducted in each of Example 3 and Comparative Example 2, and the average value and standard error of the tension were calculated for each example.

(Test results)

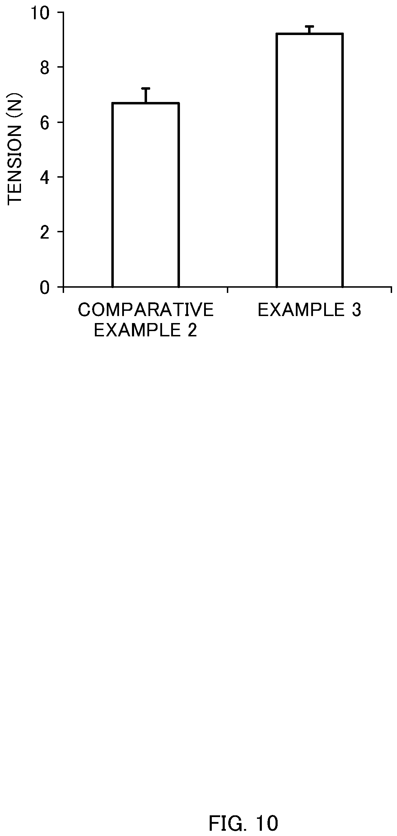

Test results are illustrated in Fig. 10. As compared to Comparative Example 2, the tension increased in Example 3, which indicated that the scleral rigidity was improved. This result indicates that a photo-sensitive substance and ultraviolet light cause cross-linking of collagen fibrils in the sclera. It was also indicated that the use of an optical probe including such a planar emitter as described above improves the scleral strength over a wide region with a single irradiation.

<Cross-linking rate of collagen>

In the field of ophthalmology, if the rigidity of the sclera is increased by cross-linking of scleral collagen fibrils, the axial elongation of the eye may be reduced, and medical conditions such as intense myopia and pathological myopia may be treated, alleviated or prevented. The following tests were conducted to demonstrate the extent to which cross-linking of collagen fibrils is caused by a light-sensitive substance and ultraviolet light. In addition, effects of the presence of air on the chemical reaction were also evaluated.

(Test method and measurement method)

Step 1) Pieces of collagen fibril sheet each weighing 5 mg (Nippi, Inc., product code 892201) were immersed in 0.25% riboflavin-5'-phosphate solution for 3 minutes.

Step 2) The pieces of collagen fibril sheet were exposed to ultraviolet light (wavelength: 365 nm, intensity: 3 $mW/cm^2$) using an ultraviolet light source (Optocode Corp., LED365-SPT/L) for 30 minutes. Then, the pieces directly exposed to ultraviolet light were taken as Example 4, and those exposed to ultraviolet light after a cover glass (0.12-0.17 mm thick) was placed thereon to prevent them from contacting air were taken as Example 5.

Step 3) The pieces were immersed in 1 mL of 0.1% picrylsulfonic acid solution at 40°C for 2 hours. Then, 50 $\mu$L of the solution was taken and diluted with water for injection by factors of 20. The absorbance of this diluted solution at 345 nm was measured with an absorbance plate reader (SPECTRAmaxPLUS384, Molecular Devices).

Examples 4 and 5, which are examples, were subjected to the above steps 1) to 3). The pieces of sheet subjected to only the above step 3) were taken as a control group. The cross-linking rates of collagen (%) in Examples 4 and 5 were calculated using the following equation.

$$\text{Cross-linking rate of collagen (\%) = 100 x ([absorbance of control group] - [absorbance of example]) / ([absorbance of control group])}$$

Two trials were conducted in each of Examples 4 and 5, and the average of the cross-linking rates of collagen in each group was calculated.

(Test results)

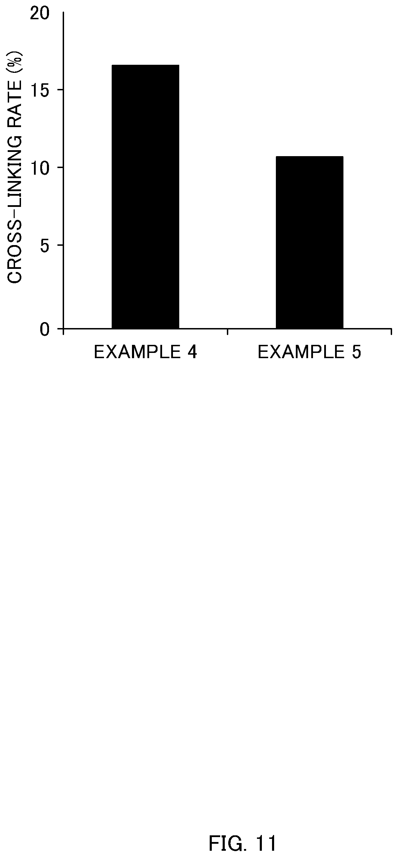

Test results are given in Fig. 11. In both Examples 4 and 5, the progress of cross-linking of collagen fibrils was observed.

It was also demonstrated that the cross-linking reaction of collagen fibrils progressed more in Example 4, which was exposed to ultraviolet light in contact with air, than in Example 5, which was exposed to ultraviolet light without air contact.

These results suggested that ultraviolet irradiation in the presence of air is more desirable for cross-linking of scleral collagen fibrils. It is thus believed that, by virtue of the supply of air, cross-linking of collagen fibrils can be caused more effectively when a medical procedure is applied to the posterior pole of the eye or it surroundings, which is not in contact with air.

[Reference Signs List]

| | |
|---|---|
| 1: | lighting system |
| 2: | light source device |
| 3, 4, 5, and 6: | optical probe |
| 3a: | insertion portion |
| 3b: | handle portion |
| 3c: | cable portion |
| 3d: | light guide plug |
| 32: | linear light guide |
| 33: | planar emitter |
| 33a: | connecter portion |
| 33b: | disk portion |
| 331: | region with a different refractive index |
| 34: | reflective surface |
| 341: | step portions |
| 342: | flat portions |
| 35: | emitting surface |
| 36: | reflective structure |
| 36a: | inner surface |
| 37: | protrusion |
| 371: | holes |
| 73: | planar emitter |
| 731a, 731b: | region with a different refractive index |
| 732: | light incident surface |
| 74: | reflective surface |
| 75: | emitting surface |
| 8: | photodetector |

**Claims**

1. An optical probe comprising:

    a linear light guide to guide light from a light source device;
    a planar emitter to be connected to a tip portion of the linear light guide, to emit the light;
    a reflective surface provided in a part of the planar emitter, to reflect the light having entered the planar emitter; and
    an emitting surface provided so as to face the reflective surface, to allow the light reflected by the reflective surface to be emitted from a region of the reflective surface.

2. The optical probe according to claim 1, wherein a distance between the emitting surface and the reflective surface in the planar emitter decreases with distance from the tip portion of the linear light guide.

3. The optical probe according to claim 1 or 2, wherein the reflective surface

    includes a plurality of step portions each having an angle with respect to the emitting surface, and a plurality of flat portions each being provided between step portions adjacent to each other of the plurality of step portions, and is in a form of a staircase in which the plurality of step portions and the plurality of flat portions are alternately continuous.

4. The optical probe according to claim 3, wherein the plurality of step portions each have an angle with respect to the emitting surface of 25 degrees or more and 65 degrees or less.

5. The optical probe according to claim 3 or 4, wherein the plurality of step portions and the plurality of flat portions are provided concentrically.

6. The optical probe according to any one of claims 1 to 5, wherein the planar emitter is substantially disk-shaped.

7. The optical probe according to any one of claims 1 to 6, wherein the reflective surface has a reflective film formed thereon.

8. The optical probe according to any one of claims 1 to 7, wherein

   a reflective structure covers the reflective surface and has a surface facing the reflective surface, and
   the surface facing the reflective surface is subjected to a reflective treatment, to reflect light having passed through the reflective surface, toward inside of the planar emitter.

9. The optical probe according to any one of claims 1 to 8, wherein the linear light guide is an optical fiber or an optical fiber bundle.

10. The optical probe according to any one of claims 1 to 9, wherein the planar emitter includes a region with a refractive index different from a refractive index or refractive indexes of the planar emitter other than the region, the region being provided on an optical path between a position at which the tip portion of the linear light guide is to be connected to the planar emitter and the reflective surface.

11. The optical probe according to any one of claims 1 to 10, further comprising:
    a protrusion protruding from the emitting surface, the protrusion having a plurality of holes communicating with outside.

12. The optical probe according to any one of claims 1 to 11, wherein an illuminated site is a site in an organism.

13. The optical probe according to claim 12, wherein the light is light to induce a chemical reaction at the site in the organism.

14. The optical probe according to claim 12 or 13, wherein the light is ultraviolet light to treat the site to which a photo-sensitive substance is administered, in the organism.

15. The optical probe according to any one of claims 12 to 14, wherein the site in the organism is an eyeball, and the emitting surface has a predetermined area such that the emitting surface can be placed along a sclera of the eyeball.

FIG. 1

FIG. 2

EP 3 915 528 A1

FIG. 3A

FIG. 3B

4

FIG. 4

FIG. 5A

FIG. 5B

6

FIG. 6A

6

FIG. 6B

FIG. 7A

FIG. 7B

FIG. 8A

73

732

R=0.3mm

1.2mm

731a

Y

X

FIG. 8B

73

732

R=0.3mm

R=0.1mm

1.2mm

731b

Y

X

FIG. 8C

73

732

1.2mm

Y

X

FIG. 9

EXAMPLE 1

EXAMPLE 2

COMPARATIVE EXAMPLE 1

RESULT OF UV PROBE SIMULATION (2)
CENTRAL CROSS-SECTION (PERPENDICULAR TO OPTIACL AXIS)

RESULT OF UV PROBE SIMULATION (2)
CENTRAL CROSS-SECTION (PARALLEL TO OPTIACL AXIS)

RESULT OF UV PROBE SIMULATION (3)
CENTRAL CROSS-SECTION (PERPENDICULAR TO OPTIACL AXIS)

RESULT OF UV PROBE SIMULATION (3)
CENTRAL CROSS-SECTION (PARALLEL TO OPTIACL AXIS)

RESULT OF UV PROBE SIMULATION (1)
CENTRAL CROSS-SECTION (PERPENDICULAR TO OPTIACL AXIS)

RESULT OF UV PROBE SIMULATION (1)
CENTRAL CROSS-SECTION (PARALLEL TO OPTIACL AXIS)

IRRADIANCE (W/cm2)

FIG. 10

FIG. 11

## INTERNATIONAL SEARCH REPORT

| International application No. |
| --- |
| PCT/JP2020/002348 |

### A. CLASSIFICATION OF SUBJECT MATTER

A61F 9/007(2006.01)i; G02B 6/00(2006.01)i; F21V 8/00(2006.01)i; A61N 5/06(2006.01)i
FI: A61N5/06 B; A61F9/007 180; F21V8/00 330; G02B6/00 326

According to International Patent Classification (IPC) or to both national classification and IPC

### B. FIELDS SEARCHED

Minimum documentation searched (classification system followed by classification symbols)
A61F9/007; G02B6/00; F21V8/00; A61N5/06

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 5487470 B2 (SONY CORP.) 07.05.2014 (2014-05-07) paragraphs [0015]-[0061], fig. 2-6 | 1-2, 6-9, 12-15 |
| Y | paragraphs [0015]-[0061], fig. 2-6 | 3-5, 10-11 |
| Y | US 7493010 B1 (EMERGING DISPLAY TECHNOLOGIES CO., LTD.) 17.02.2009 (2009-02-17) column 2, line 28 to column 4, line 8, fig. 1-5 | 3-5 |
| Y | JP 2004-241237 A (SHARP CORP.) 26.08.2004 (2004-08-26) paragraphs [0034]-[0050], fig. 6 | 10 |
| Y | JP 2015-530202 A (UNIVERSITAT LEIPZIG) 15.10.2015 (2015-10-15) paragraphs [0072]-[0087], fig. 2-5 | 11 |
| A | US 5005108 A (LUMITEX, INC.) 02.04.1991 (1991-04-02) column 7, line 9 to column 8, line 68, fig. 15-21 | 1-2, 7-9, 12-13 |

☒ Further documents are listed in the continuation of Box C.   ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 March 2020 (16.03.2020) | 31 March 2020 (31.03.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2020/002348

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2009-526368 A (RPC PHOTONICS, INC.) 16.07.2009 (2009-07-16) paragraphs [0022]-[0024], [0113], [0161]-[0165], fig. 1-2, 38, 69 | 1-9 |

Form PCT/ISA/210 (continuation of second sheet) (January 2015)

International application No.

PCT/JP2020/002348

| Patent Documents referred in the Report | Publication Date | Patent Family | Publication Date |
|---|---|---|---|
| JP 5487470 B2 | 07 May 2014 | (Family: none) | |
| US 7493010 B1 | 17 Feb. 2009 | (Family: none) | |
| JP 2004-241237 A | 26 Aug. 2004 | (Family: none) | |
| JP 2015-530202 A | 15 Oct. 2015 | US 2015/0257928 A1 paragraphs [0096]-[0111], fig. 2-5 CN 104936563 A KR 10-2015-0095628 A WO 2014/056895 A1 | |
| US 5005108 A | 02 Apr. 1991 | US 5136480 A | |
| JP 2009-526368 A | 16 Jul. 2009 | US 2007/0189701 A1 paragraphs [0098]-[0100], [0152], [0196]-[0201], fig. 1-2, 38, 69 CA 2641961 A1 CN 101421557 A CN 102620242 A KR 10-2009-0015884 A US 2009/0297113 A1 US 2010/0027288 A1 WO 2007/095049 A2 | |

Form PCT/ISA/210 (patent family annex) (January 2015)

26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6309654 B
- JP 6336130 B

- JP 5487470 B

**Non-patent literature cited in the description**

- **J CATARACT.** *Refract Surg.,* 2004, vol. 30 (3), 689-695